# EUROPEAN PATENT APPLICATION

(11) **EP 2 526 768 A1**
(43) Date of publication of application: **28.11.2012**
(21) Application number: 12382199.3
(22) Date of filing: 24.05.2012
(51) Int. Cl.: A01N 1/02

(54) **Extender for a semen suspension**

(30) Priority: 24.05.2011 ES 201130842
(71) Applicant: Iberica De Reproduccion Asistida, S.L., 08540 Centelles (Barcelona) (ES); Gil Pascual, Javier, 40002 Segovia (ES)
(72) Inventor: Gil Pascual, Javier, 40002 Segovia (ES)
(74) Representative: Carpintero Lopez, Francisco

(57) **Abstract**

The present invention belongs to the veterinary field, and particularly to the field of assisted reproduction. The invention more specifically consists of a semen extender comprising alginate and the use thereof for sperm preservation and for increasing the percentage of offspring of a certain sex.

## Description

### Field of the Invention

The present invention belongs to the field of artificial insemination of animals. The invention more specifically consists of a semen extender comprising alginate and the use thereof for sperm preservation and for increasing the percentage of offspring of a certain sex.

### Background of the Invention

One of the biggest problems today in artificial insemination techniques is the preservation of sperm (or semen) and its fertilizing power until the moment of insemination arrives. To solve this problem a medium referred to as "extender" in which the sperm is diluted immediately after being collected, is commonly used. It is therefore preserved until the moment of insemination.

The preservation time that can be obtained in the presence of an extender depends on the species in question and on the preservation conditions, and particularly on the type of extender chosen; said time generally ranges from a few hours to a few days. In some species, this time can be prolonged by means of freezing the sperm; however, this technique also destroys or inactivates a considerable number of spermatozoa, and therefore lowers sperm fertility. There are also considerable differences in preservation efficacy by using the freezing process according to the animal species in question. In this regard, artificial insemination in pigs is particularly problematic because even though the freezing technique has meant good standardization of the preservation methods in other domesticated species, it is virtually inapplicable with porcine seminal material. The peculiar particularities of porcine spermatozoon make it very sensitive to cold shock, causing a sperm viability alteration. This sensitivity to cold shock in practice means that the semen samples must be preserved at 15-20°C because a reduced storage temperature limits the viability of the semen samples (Paulenz *et al.,* 2000).

The present invention relates to the addition of alginate at between 0.25 g/L and 10 g/L conventional in semen extenders (both of boars and of other animal species) to improve the survival of the spermatozoa and their behavior in the fertilization with a view to artificial insemination or any other assisted reproduction practice.

There are many studies disclosing the use of alginate as a semen extender, for example in the caprine, ovine, bovine, and equine species. However, they all require the encapsulation process to preserve the semen (ES2202723; EP0922451; Herrler et al., 2006). Surprisingly, the present invention describes the use of alginate in solution at a concentration between 0.25 g/L and 10 g/L which allows preserving the sperm without needing to carry out the encapsulation process. In the extender with alginate of the present invention, contact between sperm cells is reduced or eliminated, so said cells remain either partially or completely in suspension, favoring contact with the extender and thereby the preservation, viability and fertilizing capacity, without subjecting them to chemical reactions required for encapsulation.

In the terms of the present invention, sperm or semen is understood as the set of spermatozoa and fluid substances produced in the male genital organs of animals and of the human species. In the terms of this invention, conventional semen extender is understood as the aqueous solution that allows increasing the seminal volume (higher number of semen doses per ejaculate) and preserving the functional characteristics of the sperm cells and maintaining the suitable fertility level. The spermatozoa are found in the seminal plasma which provides the nutrients necessary for maintaining high metabolic activity necessary for the sperm transport process through the female genital tract. To perform its function, the extender must provide the nutrients necessary for the metabolic maintenance of the sperm cell, for controlling the pH of the medium, osmotic pressure and microbial growth inhibition. To that end, most extenders comprise sugars, such as glucose, galactose, fructose, ribose or trehalose; NaCl or KCI salts; bicarbonate, TRIS and/or HEPES. They can additionally contain chelating agents, such as EDTA, antibiotics and/or BSA.

Conventional extenders for boar and rabbit semen are mixtures of chemical products aimed at producing an environment where spermatozoa can find the energy and osmotic pressure, ionic strength and pH conditions allowing them to maintain their fertilizing capacity for a specific time period.

The following table (Table 1) shows the published composition of several standard extenders for porcine semen (Gadea J., 2003).

**Table 1.- Composition (in g/L) of several standard extenders for porcine semen for one liter of distilled water.**

| | IVT | Kiev | BTS | Zorle SCO | MR-A | ZOR PVA | Reading | Moden a | Androhe p |
|---|---|---|---|---|---|---|---|---|---|
| Glucose | 3 | 60 | 37 | 11.5 | + | 11.5 | 11.5 | 25^{a} | 26 |
| Sodium citrate | 24.3 | 3.7 | 6.0 | 11.7 | + | 11.65 | 11.65 | 6.90 | 8.0 |
| EDTA | | 3.7 | 1.25 | 2.3 | + | 2.35 | 2.35 | 2.25 | 2.4 |
| Sodium bicarbonate | 2.4 | 1.2 | 1.25 | 1.25 | + | 1.75 | 1.75 | 1.00 | 1.2 |
| Potassium chloride | 0.4 | | 0.75 | | - | | 0.75 | | |
| Acetylcysteine | 0.05 | | | | | | | | |
| HEPES | | | | | | | | | 9.0 |
| BSA | | | | 5.0 | + | | | 3.00 | 2.5 |
| TRIS | | | | 6.5 | - | 5.5 | 5.5 | 5.65 | |
| Citric acid | | | | 4.1 | - | 4.1 | 4.1 | 2.00 | |
| Cysteine | | | | 0.1 | + | 0.7 | 0.7 | 0.05 | |
| Trehalose | | | | | | | 1 | | |
| PVA | | | | | | 1 | 1 | | |
| Potassium acetate | | | | | + | | | | |
| MOPS | | | | | + | | | | |
| Osmotic pressure (mOsm) | 290 | 380 | 330 | 240 | 290 | 275 | 300 | 282 | 309 |
| pH | | 7.2 | 7.2 | | 6.9 | | | 6.9 | 6.8 |

| | | | | | | | | | |
|---|---|---|---|---|---|---|---|---|---|
| - ^{a}: glucose monohydrate | | | | | | | | | |

The following table (Table 2) likewise shows the published composition of the DLBR extender for rabbit semen (ES2190723).

**Table 2.-Composition of the DLBR extender for rabbit semen per liter of distilled water.**

| Compound | Concentration |
|---|---|
| Tris(hydroxymethyl)amino methane | 0.250 M |
| Citric acid | 0.0828 M |
| Glucose | 0.050 M |
| EDTA | 0.100 mM |
| Dihydrostreptomycin sulfate | 1.250 mg |
| Penicillin G sodium | 300,000 IU |
| Penicillin G procaine | 700,000 IU |

There are different conventional semen extenders on the market, and among them, Import-Vet S.A. markets two types of extender for boar semen (ND-4 and ND-5) and one for rabbit semen (ND-C7). Porcine ND-4 is an extender suitable for maintaining sperm for 3 to 4 days, porcine ND-5 is suitable for maintaining it for 6 to 7 days, and ND-C7 for rabbit keeping is suitable for maintaining it for 1 to 2 days.

The following table (Table 3) shows the qualitative composition of the three extenders marketed by Import-Vet S.A. (ND-4, ND-5, ND-C7), where the (+) symbol indicates the presence of the compound in question in the extender (Gil Pascual J., results not published).

**Table 3.- Qualitative composition of extenders ND-4, ND-5 and ND-C7.**

| | ND-4 | ND-5 | ND-C7 |
|---|---|---|---|
| Glucose | + | + | + |
| Sodium citrate | + | + | + |
| EDTA | + | + | |
| Sodium bicarbonate | + | + | + |
| Potassium chloride | + | + | + |
| Acetylcysteine | | + | + |
| MOPS | | + | + |
| Osmotic pressure (mOsm) | 300 | 293 | 276 |
| pH | 7.20 | 6.90 | 6.40 |

When conventional extenders (without alginate) are used, over time the spermatozoa are deposited on the bottom of the vessel used for containing the semen dose, whether it is a bottle, a heat-sealed tube or a blister. The compaction generated reduces contact of the spermatozoa with the extender, complicates the transport of nutrients into the cell, alters metabolism and favors the formation of clumping, which all together alter the state of the sperm membrane and reduce maintenance of the fertilizing power of the spermatozoa over time.

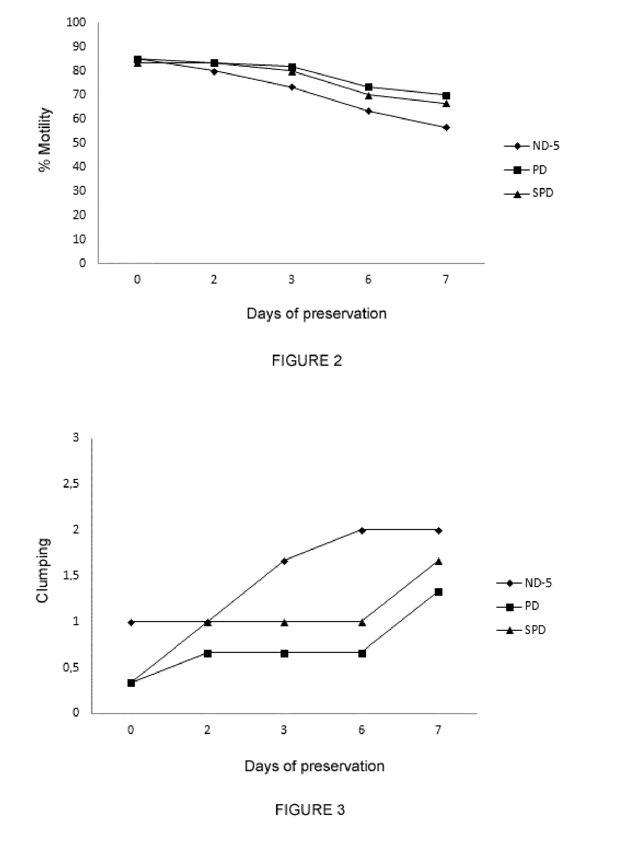

Any conventional extender, among others those manufactured by Import-Vet S.A. (ND-4, ND-5, ND-C7), to which alginate has been added at a concentration between 0.25 and 10 g/L, object of the present invention, improves the preservation of sperm and its fertilizing power. The extender of the present invention has greater density, which varies according to the alginate concentration (the higher the alginate concentration the greater the density). It therefore reduces or prevents decantation of the spermatozoa which, since they are kept separate from one another, maintain contact with the extender, allowing the metabolic exchange with the medium and reducing or preventing the formation of clumping over time. The characteristics of the sperm membrane are therefore maintained for a longer time, improving the sperm preservation and fertilization capacity. Motility (Figure 2) and spermatozoa aggregation (Figure 3) are included among other features improved by using the extender of the present invention.

Finally, various studies demonstrate that the normal separation of sexes in both natural mating and in artificial insemination is close to 50/50 (Soede *et al.,* 2000). However, by using the extender object of the present invention the percentage in the offspring of one sex or another can be increased according to whether one wants more males or more females. The male spermatozoa carrying Y chromosomes, and the female spermatozoa carrying X chromosomes have certain different characteristics, such as their weight and movement capacity for example (Table 4).

**Table 4.- Different parameters between X and Y spermatozoa.**

| Parameter | Difference |
|---|---|
| DNA | X greater than Y |
| Size | X greater than Y |
| Motility | Y faster than X |
| *F-Body* | Heterochromatin in Y |
| Surface charge | X migrates towards cathode |

The extender of the present invention allows the spermatozoa to partially separate into different layers over time. Due to the differential characteristics of the female and male spermatozoa, more female spermatozoa are in the top layer and more male spermatozoa are in the bottom layer. Selecting one layer or another favors the percentage of offspring being larger for one sex or the other. More female animals are obtained with the spermatozoa taken from the top layer and more male animals are obtained with the spermatozoa from the bottom layer.

### Object of the Invention

An object of the present invention consists of a semen extender comprising alginate. Another object of the present invention is the application of said solution for better sperm preservation over time and for increasing the percentage of the offspring of a certain sex.

### Brief Description of the Drawings

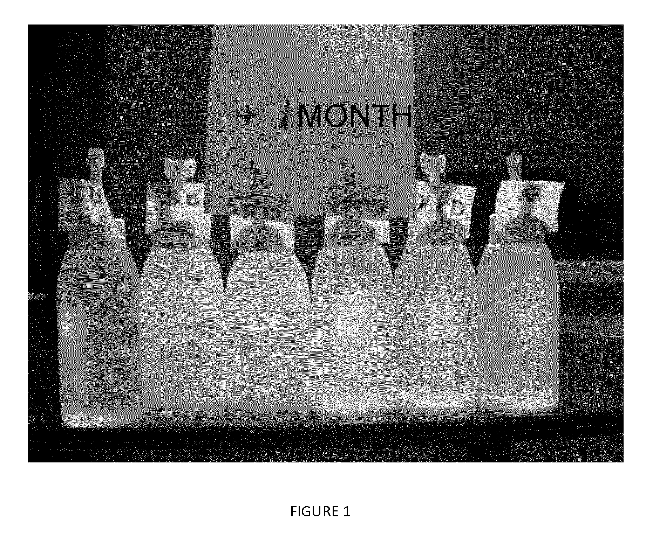

Figure 1 shows several bottles with the same amount of diluted semen in different extenders: one with conventional extender (N) (in this case extender ND-5 by Import-Vet S.A.) and four in which different concentrations of alginate have been added to extender ND-5 by Import-Vet, hereinafter referred to as: SD, PD, XPD and MPD. SD refers to Semi Dense and contains between 5.00 and 10.00 g/L of alginate; PD refers to Scarcely Dense and contains between 2.5 and 4.9 g/L of alginate; MPD refers to Very Scarcely Dense and contains between 1.25 and 2.4 g/L of alginate; and XPD refers to Extra Scarcely Dense and contains between 0.75 and 1.24 g/L of alginate. There is also a bottle with conventional extender and without semen (SD without S). Less decantation occurs at higher alginate concentration. In SD and PD it can be seen how the vast majority of the spermatozoa remain floating despite the fact that a month has transpired since the production of the doses. Although the spermatozoa have decanted in XPD and MPD, the pellet formed is much less dense and wider than in the dose produced with the conventional extender (N).
Figure 2 shows a graphical depiction of the percentage of motility (%) of the sperm over time (days). The sperm is diluted in conventional extender (ND-5), Scarcely Dense extender (PD, extender ND-5 with alginate at a concentration between 2.5 and 4.9 g/L) and Extra Scarcely Dense extender (XPD, extender ND-5 with alginate at a concentration between 0.75 and 1.24 g/L). It is observed that the addition of alginate keeps motility of the spermatozoa higher over time than the conventional extender.
Figure 3 shows a graphical depiction of the percentage of clumping (%) of the sperm over time (days). The sperm is diluted in conventional extender (ND-5), Scarcely Dense extender (PD) and Extra Scarcely Dense (XPD). It is observed that the addition of alginate delays clumping of the spermatozoa until the sixth day of preservation, unlike the conventional extender, where increased clumping is observed from the second day of storage. Therefore, clumping is reduced by adding alginate in comparison with the clumping observed with the conventional extender.

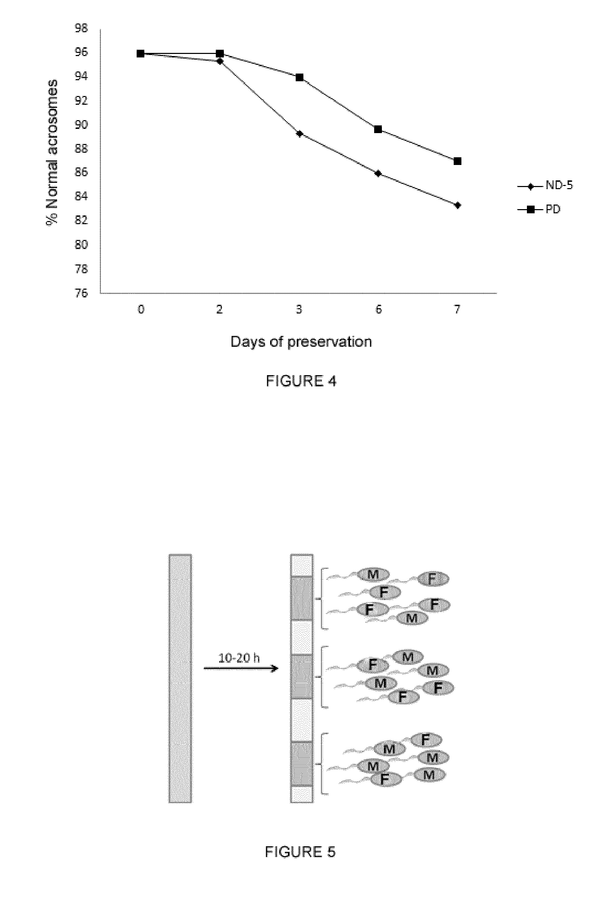

Figure 4 shows a graphic depiction of the percentage of normal acrosomes (%) in the spermatozoa over time (days), the sperm being diluted in conventional extender (ND-5) and in Scarcely Dense extender (PD). It is observed that the addition of the composition object of this invention keeps the percentage of normal acrosomes in the spermatozoa higher over time.
Figure 5 depicts the enrichment of female spermatozoa (H) in the top layer and of male spermatozoa (M) in the bottom layer after leaving the diluted semen in extender with alginate in tube-shaped bags at rest for between 10 and 20 hours.

### Detailed Description of the Invention

The technical object of the present invention is a semen extender characterized in that it comprises alginate at a concentration of 0.25 to 10 g/L, and wherein the spermatozoa are in suspension. In the context of the present invention, it is understood that the spermatozoa are in suspension when they are maintained in a fluid, in this case the extender with alginate of the invention, without precipitating to the bottom.

In a particular embodiment of the present invention, the extender comprises sodium, barium or calcium alginate. In a more particular embodiment, the extender comprises sodium alginate.

The alginate concentration in the extender of the invention ranges between 0.25 and 10 g/L of extender. In a particular embodiment, the extender comprises between 5.00 and 10.00 g/L of alginate resulting in a Semi Dense extender (SD). In another particular embodiment, the extender comprises between 2.5 and 4.9 g/L of alginate resulting in a Scarcely Dense extender (PD). In another particular embodiment, the extender comprises between 1.25 and 2.40 g/L of alginate resulting in a Very Scarcely Dense extender (MPD). In another particular embodiment, the extender comprises between 0.75 and 1.24 g/L of alginate resulting in an Extra Scarcely Dense extender (XPD).

In a particular embodiment of the present invention, alginate is added to a conventional semen extender, such as MR-A (Table 1), Acromax (manufactured by GVP-Gestion Veterinaria Porcina), Duragen (manufactured by Magapor), Diluyente Esperma Porcino SP Veterinaria (manufactured by SP Veterinaria), Safe-cell (manufactured by IMV-Technologies), Androhep (Table 1), ND-4, ND-5 and ND-C7 (Table 3). In a preferred embodiment, the alginate is added to ND-4, ND-5 or ND-C7.

Another object of the present invention is the use of the extender of the invention to reduce sperm clumping and favor sperm preservation as well as to improve reproductive outcomes in the artificial insemination of livestock, and more particularly of pigs and rabbits. In another particular embodiment, an object of the present invention is the use of the extender of the invention for separating different-sex spermatozoa (Y or X), and for increasing the percentage of offspring of a certain sex.

Finally, another object of the present invention is also a method to increase the percentage of female or male spermatozoa, characterized in that it comprises the following steps:
a) obtaining semen from the male animal,
b) diluting said semen in the extender object of the invention until reaching a dilution ratio between 1/2 and 1/10 ejaculate/extender of the invention,
c) filling tube-shaped plastic bags between 35 to 45 mm wide and 350 and 450 mm long with said diluted semen,
d) leaving the bag at rest in the vertical position for 10 and 20 hours at between 19 and 25ºC so that the spermatozoa separate into different layers,
e) clamping the tubes separating the different layers,
f) obtaining spermatozoa from the top layer to increase the number of female animals and from the bottom layer to increase the number of male animals.

Finally, another object of the present invention is a method of increasing the percentage of the offspring of a certain sex, characterized in that it comprises the following steps:
i) carrying out the method of increasing the percentage of male or female spermatozoa described above,
ii) artificially inseminating with the semen obtained in step i).

A series of examples illustrating the present invention but not limiting the scope thereof in any way is provided below.

### Example 1.- Preservation and maintenance of the fertilizing potential of the spermatozoa

Studies on the motility, aggregation and acrosomes of the sperm diluted in different extenders (with and without sodium alginate at different concentrations) were carried out as follows:

Motility: Two different operators in two different laboratories received the same semen samples. Slides containing a drop of semen diluted in the different extenders (with and without alginate) are arranged on a plate heated to 39ºC; after a few seconds the sample is covered with an also tempered cover slip and observed under the microscope at 200 and 400 magnifications. The percentage of spermatozoa with movement is subjectively calculated.

Clumping: Following the same method as for the study on motility, the degree of clumping is rated from 0 to 3.

Acrosomes: 1 ml of diluted semen is mixed in each of the different extenders with 1 ml of a formol saline solution that kills and fixes the spermatozoa. A small drop of this mixture is deposited on a slide under cold conditions, covered with a cover slip, and the percentage of normal acrosomes is observed at 1500 magnifications in a phase contrast microscope using immersion oil.

The results obtained are depicted in Figures 2, 3 and 4, where it can be seen how the motility of the spermatozoa remains higher over time in the extenders with alginate of the present invention. It can also be seen how clumping virtually does not increase until the sixth day of preservation in the dense extenders when it does increase in the conventional extender after the second day. Finally, it can be seen that the percentage of normal acrosomes remains higher in the spermatozoa diluted in extender with alginate of the present invention than in those with conventional extender.

Artificial inseminations were performed with the sperm thus preserved (Manual de inseminación artificial, Kubus S.A.) and the results were analyzed based on the offspring obtained. Mummified births (Mum), total births (TB), live births (LB), stillbirths (SB) and the positive pregnancy diagnosis by means of ecography (Eco+) were quantified. The "mean" refers to the mean per litter, i.e., total number of live births/number of deliveries (Table 5).

**Table 5.- Fertility and prolificacy after artificial insemination (Al).**

| WEEK covered | ND5 | | | XPD | | | | |
|---|---|---|---|---|---|---|---|---|
| | No. Al | Eco + | % | No. Al | Eco + | % | | |
| 32 | 8 | 5 | 62.50 | 12 | 10 | 83.33 | | |
| 33 | 8 | 8 | 100.00 | 11 | 10 | 90.91 | | |
| 34 | 9 | 9 | 100.00 | 8 | 8 | 100.00 | | |
| 35 | 8 | 7 | 87.50 | 10 | 10 | 100.00 | | |
| 36 | 5 | 3 | 60.00 | 7 | 7 | 100.00 | | |
| 37 | 9 | 6 | 66.67 | 12 | 11 | 91.67 | | |
| Total | 47 | 38 | 80.85 | 60 | 56 | 93.33 | | |
| | | | | | | | | |

| WEEK covered | ND5 | | | | XPD | | | |
|---|---|---|---|---|---|---|---|---|
| | Deliveries | LB | SB | TB | Deliveries | LB | SB | TB |
| 32 | 4 | 37 | 4 | 41 | 10 | 112 | 10 | 122 |
| 33 | 8 | 86 | 6 | 92 | 9 | 101 | 8 | 109 |
| 34 | 9 | 97 | 10 | 107 | 8 | 88 | 10 | 98 |
| 35 | 7 | 82 | 8 | 90 | 9 | 100 | 11 | 111 |
| 36 | 3 | 32 | 4 | 36 | 6 | 64 | 7 | 71 |
| 37 | 4 | 48 | 3 | 51 | 8 | 93 | 6 | 99 |
| Total | 35 | 382 | 35 | 417 | 50 | 558 | 52 | 610 |
| Mean | | 10.91 | 1.00 | 11.91 | | 11.16 | 1.04 | 12.20 |

This table shows that fertility and prolificacy are greater when a dense extender, in this case extra-Scarcely Dense, i.e., with a sodium alginate concentration between 0.75 and 1.24 g/L (XPD, Table 5) is used.

In turn, the results in rabbit keeping also demonstrate the good reproductive behaviour obtained with doses in which the semi-dense extender (ND-C7 with alginate at a concentration between 5.00 and 10.00 g/L) has been used against the conventional extender (ND-C7) (Table 6).

**Table 6.- Prolificacy after artificial insemination of rabbits.**

| | Semi-dense extender | | | | | Conventional extender | | | | |
|---|---|---|---|---|---|---|---|---|---|---|
| | Al | Positive | % | Give Birth | % | Al | Positive | % | Give Birth | % |
| Rabbits | 21 | 18 | 85.71 | 17 | 80.95 | 40 | 33 | 82.50 | 32 | 80.00 |
| Rabbits | 20 | 20 | 100 | 19 | 95.00 | 44 | 38 | 86.36 | 38 | 86.36 |
| Total | 41 | 38 | 92.68 | 36 | 87.80 | 84 | 71 | 84.52 | 70 | 83.33 |

In the control rabbits inseminated with conventional extender (ND-C7, without alginate), fertility at confirmation of pregnancy by palpation (Positive) is considerably less, 84.52% compared with 92.68% obtained with the semi-dense extender. The rate of deliveries with the conventional extender is also less, 83.33% compared with 87.80% of the rabbits inseminated with the semi-dense extender (with sodium alginate between 5.00 and 10.00 g/L).

### Example 2.- Increased percentage of offspring of a certain sex

When boar semen is suspended in the semi-dense extender (SD) object of the present invention, the spermatozoa partially separate over time into different layers, there being more female spermatozoa in the top layer and more male spermatozoa in the bottom layer (Figure 5).

The semen is collected on a small amount of dense extender, contrasted to determine its quality and know how many doses can be made, more dense extender is added until achieving a sufficient level of dilution assuring semen preservation for at least 24 hours (dilution between 1/2 and 1/10 ejaculate/extender of the invention). Tube-shaped plastic bags between 35 to 45 mm wide and between 350 to 450 mm long (decantation bag) are filled with said diluted semen. The bags are left at rest in the vertical position for 10 to 20 hours, at a temperature between 19 and 25ºC so that the female and male spermatozoa separate into different layers (fractions). After the clear separation of the fractions, the tubes are clamped to separate their content into a fraction enriched with female spermatozoa (top layer), another mixed fraction (middle layer) and another fraction enriched with male spermatozoa (bottom layer). Each fraction is weighed and rediluted with conventional extender until reaching the volume necessary for being able to produce the total number of doses calculated during contrasting.

Once on the farm, the doses are preserved at 15ºC up to a maximum of 6 days. The pigs are inseminated 2 or 3 times every 12 or 24 hours and according to the duration of the heat cycle with the extender obtained from the different top, middle or, bottom layers. Representative results are shown in Table 7.

**Table 7.- Increased percentage of a determined sex in the offspring.**

| **Fraction** | **AI** | **Birth** | **MALE** | **FEMALE** | **Total** | **Mean TB** |
|---|---|---|---|---|---|---|
| | | | | | Difference | |
| Top | 14 | 12 | 64 | 96 | 160 | 13.33 |
| % | | 85.7 | 40.0 | 60.0 | 20.0 | |
| | | | | | | |
| Middle | 5 | 4 | 23 | 31 | 54 | 1350 |
| % | | 80,0 | 426 | 574 | 148 | |
| | | | | | | |
| Bottom | 14 | 14 | 100 | 90 | 190 | 13.57 |
| % | | 100 | 52.6 | 47.4 | -5.3 | |
| | | | | | | |
| All | 33 | 30 | 187 | 217 | 404 | 13.47 |
| % | | 90.9 | 46.3 | 53.7 | 7.4 | |

When the three groups are consolidated, the overall results show that the mean of the 30 pigs that birth is 46.3% males and 53.7% females so between both groups there is only a 7.4% difference between the amount of males and females.

However in the group inseminated with the top fraction, the percentage of males is 40% and females is 60% so the difference between sexes increases up to 20%. A progressive reduction of the percentage of females from the top fraction to the middle and bottom fraction with 60%, 57.4% and 47.4%, respectively, is also observed.

Therefore, the separation of the number of male and female spermatozoa in the different fractions of the decantation bag is progressive and real, and therefore the use of alginate in conventional extenders allows favoring obtaining males or females according to the fraction taken for insemination.

### Literature

EP0922451 (16/06/1999)
ES2190723 (01/08/2003)
Gadea J., 2003. Los diluyentes de inseminación artificial porcina. Review. Spanish Journal of Agricultural Research. (2):17-27.
Herrler A., Eisner S, Bach V, Weissenborn U, Beier HM., 2006. Cryopreservation of spermatozoa in alginic acid capsules. Fertil. Steril. 85(1):208-13.
Kubus S.A. (Legal Deposit: M 8929-1999), Manual de inseminación artificial.
Paulenz H., Kommisrud E., Hofmo P.O., 2000. Effect of Long-Term storage at different temperatures on quality of liquid boar semen. Reprod. Dom. Anim. 35, 83-85.
Soede NM, Nissen AK, Kemp B. Timing of insemination relative to ovulation in pigs: effects on sex ratio of offspring. Theriogenology, 2000 Mar 1;53(4):1003-11.

## Claims

1. Semen extender, **characterized in that** it comprises alginate at a concentration of 0.25 to 10 g/L, and wherein the spermatozoa are in suspension.

2. Extender according to the preceding claim, **characterized in that** the alginate is barium, calcium or sodium alginate.

3. Extender according to the preceding claim, **characterized in that** the alginate is sodium alginate.

4. Extender according to any of claims 1 to 3, **characterized in that** it comprises between 5 and 10 g/L of alginate.

5. Extender according to any of claims 1 to 3, **characterized in that** it comprises between 2.5 and 4.90 g/L of alginate.

6. Extender according to any of claims 1 to 3, **characterized in that** it comprises between 1.25 and 2.40 g/L of alginate.

7. Extender according to any of claims 1 to 3, **characterized in that** it comprises between 0.75 and 1.24 g/L of alginate.

8. Use of the extender according to any of claims 1 to 7 to reduce sperm clumping and favor sperm preservation.

9. Use according to any of claims 1 to 7 to improve the reproductive outcomes in the artificial insemination of livestock.

10. Use according to claim 9 for the artificial insemination of pigs and rabbits.

11. Use of the extender according to any of claims 1 to 7 for separating different-sex spermatozoa.

12. Use of the extender according to any of claims 1 to 7 to increase the percentage of offspring of a certain sex.

13. Method for increasing the percentage of female or male spermatozoa, **characterized in that** it comprises the following steps:
a) obtaining semen from the male animal,
b) diluting the ejaculate to between 1/2 and 1/10 ejaculate/extender according to any of claims 1 to 7,
c) filling tube-shaped plastic bags 35 to 45 mm wide and 350 to 450 mm long with said diluted semen,
d) leaving the bag at rest in the vertical position for 10 to 20 hours at a temperature between 19 and 25ºC so that the spermatozoa separate into different layers,
e) clamping the bags separating the different top, middle and bottom layers,
f) obtaining spermatozoa from the top layer to increase the number of female animals and from the bottom layer to increase the number of male animals.

14. Method of increasing the percentage of the offspring of a certain sex, **characterized in that** it comprises the following steps:
i. carrying out the method of claim 13,
ii. artificially inseminating with the semen obtained in step i).
